(19) Europäisches Patentamt
European Patent Office
Office européen des brevets

(11) Veröffentlichungsnummer: **0 158 205**
**B1**

(12) **EUROPÄISCHE PATENTSCHRIFT**

(45) Veröffentlichungstag der Patentschrift: **18.01.89**

(21) Anmeldenummer: **85103505.5**

(22) Anmeldetag: **25.03.85**

(51) Int. Cl.⁴: **C 07 D 413/06**, C 07 D 417/06, C 07 D 413/04, C 07 D 417/04, A 01 N 43/653, A 01 N 43/72

(54) **(1,2,4-Triazol-1-yl)-methyl-carbinole.**

(30) Priorität: **07.04.84 DE 3413173**

(43) Veröffentlichungstag der Anmeldung:
**16.10.85 Patentblatt 85/42**

(45) Bekanntmachung des Hinweises auf die Patenterteilung:
**18.01.89 Patentblatt 89/3**

(84) Benannte Vertragsstaaten:
**AT BE CH DE FR GB IT LI NL**

(56) Entgegenhaltungen:
**EP-A- 0 046 337**
**EP-A- 0 097 480**
**GB-A- 2 120 235**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Gajowski, Jan, Dr., Malvenweg 8, D-2900 Oldenburg (DE)**
Erfinder: **Regel, Erik, Dipl.-Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Reinecke, Paul, Dr., Steinstrasse 8, D-5090 Leverkusen 3 (DE)**

**EP 0 158 205 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 1,2,4-Triazol-1-ylmethyl-carbinole, ein Verfahren zu ihrer Herstellung und ihre Verwendung als Fungizide.

Es ist bereits bekannt, dass bestimmte heterocyclische Stickstoffverbindungen, wie beispielsweise das 6-Methyl-2-oxo-dithiolo(4,5-b)-chinoxalin (vgl. DE-B 1 100 372), oder auch bestimmte 1,2,4-Triazol-1-ylmethyl-carbinole, wie beispielsweise das 1-(4-Biphenylyl)-1-(2-fluorphenyl)-2-(1,2,4-triazol-1-yl)-ethanol oder das 1-(4'-Chlor-4-biphenylyl)-1-(4-chlorphenyl)-2-(1,2,4-triazol-1-yl)-ethanol (vergl. DE-A 2 920 374) fungizide Eigenschaften besitzen.

Die Wirkung dieser Verbindungen ist jedoch, insbesondere bei niedrigen Aufwandmengen und Konzentrationen, nicht immer in allen Anwendungsbereichen völlig zufriedenstellend.

Weiterhin sind aus der GB-A 2 120 235 und der EP-A 0 046 337 fungizid wirksame Hydroxyethylazolyl-Derivate bekannt, in denen das Carbinol-Kohlenstoffatom mit einem heterocyclischen Rest verbunden sein kann. Es werden jedoch keine Verbindungen offenbart, in denen der heterocyclische Rest für einen Heteroaromaten mit zwei verschiedenen Heteroatomen steht.

Es wurden nun neue 1,2,4-Triazol-1-ylmethyl-carbinole der allgemeinen Formel (I),

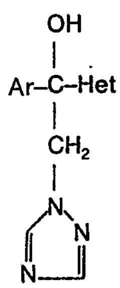

(I)

in welcher

Ar für gegebenenfalls ein- bis dreifach, gleich oder verschieden substituiertes Phenyl oder Naphthyl steht, wobei als Substituenten Fluor, Chlor, Brom, Cyano, Nitro, Methyl, Ethyl, Propyl, Butyl, Methoxy, Ethoxy, Methylthio, Trifluormethyl, Trifluormethoxy und Trifluormethylthio zu nennen sind, und

Het für einen gegebenenfalls ein- bis dreifach, gleich oder verschieden durch Methyl, Ethyl, Cyclopropyl, Cyclohexyl, Fluor, Chlor, Brom, Trifluormethyl, Chlordifluormethyl, Dichlorfluormethyl, Trichlormethyl, Dichlormethyl, Chlormethyl oder durch gegebenenfalls ein- bis dreifach substituiertes Phenyl substituierten Heterocyclus der Formel

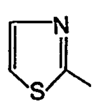 oder 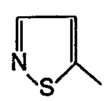 steht,

sowie deren pflanzenverträgliche Säureadditionssalze und Metallsalzkomplexe gefunden.

Weiterhin wurde gefunden, dass man die neuen 1,2,4-Triazol-1-ylmethyl-carbinole der allgemeinen Formel (I) sowie deren pflanzenverträgliche Säureadditionssalze und Metallsalzkomplexe erhält, wenn man Oxirane der Formel (II),

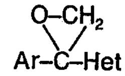

(II)

in welcher

Ar und Het die oben angegebene Bedeutung haben, mit Alkalisalzen von 1,2,4-Triazol der Formel (III),

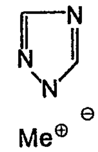

(III)

in welcher

$Me^{\oplus}$ für ein Alkalimetallkation steht,

gegebenenfalls in Gegenwart eines Verdünnungsmittels umsetzt und gegebenenfalls noch an die so erhaltenen Verbindungen der Formel (I) anschliessend eine Säure oder ein Metallsalz addiert, die zu einem pflanzenverträglichen Säureadditions-Salz oder Metallsalz-Komplex führen.

Schliesslich wurde gefunden, dass die neuen 1,2,4-Triazol-1-ylmethyl-carbinole der allgemeinen Formel (I) sowie deren pflanzenverträgliche Säureadditionssalze und Metallsalzkomplexe fungizide Eigenschaften besitzen.

Überraschenderweise zeigen die neuen 1,2,4-Triazol-1-ylmethyl-carbinole der allgemeinen Formel (I) eine bessere fungizide Wirksamkeit als die aus dem Stand der Technik bekannten Verbindungen

6-Methyl-2-oxo-1,3-dithiolo-[4,5-b]-chinoxalin bzw.
1-(4-Biphenylyl)-1-(2-fluorphenyl)-2-(1,2,4-triazol-1-yl)-ethanol oder
1-(4'-Chlor-4-biphenylyl)-1-(4-chlorphenyl)-2-(1,2,4-triazol-1-yl)-ethanol,

welches chemisch und/oder wirkungsmässig naheliegende Verbindungen sind.

Im einzelnen seien ausser den bei den Herstellungsbeispielen genannten Verbindungen die folgenden 1,2,4-Triazol-1-ylmethyl-carbinole der allgemeinen Formel (I) genannt:

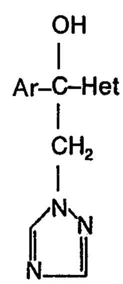

| Het | Ar |
|---|---|
| 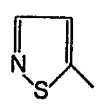 | 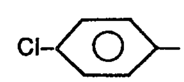 |
| 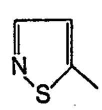 | 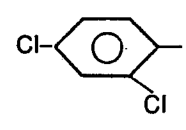 |

| Het | Ar |
|---|---|
| 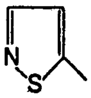 | 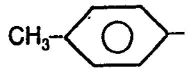 |
| 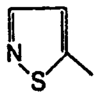 | 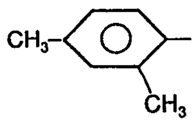 |
| 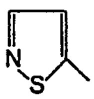 | 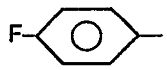 |
| 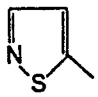 | 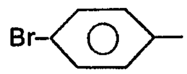 |

Verwendet man als Ausgangsstoffe beispielsweise 2-(4-Chlorphenyl)-2-thiazol-2-yloxiran und 1,2,4-Triazol-Natriumsalz, so lässt sich der Reaktionsablauf des erfindungsgemässen Verfahrens durch das folgende Formelschema darstellen:

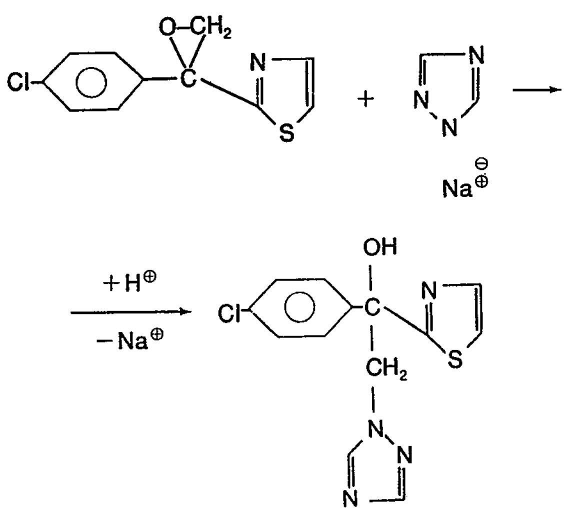


Die als Ausgangsstoffe zur Durchführung des erfindungsgemässen Verfahrens benötigten Oxirane sind durch die Formel (II) allgemein definiert. In dieser Formel (II) stehen Ar und Het für diejenigen Reste, die schon bei der Beschreibung der erfindungsgemässen Stoffe der Formel (I) für diese Substituenten genannt wurden.

Die Oxirane der Formel (II) sind noch nicht bekannt. Man erhält sie jedoch nach prinzipiell bekannten Verfahren (vergl. z.B. DE-A 3 237 400), wenn man Heterocyclen der Formel (IV),

Het–X (IV)

in welcher

Het die oben angegebene Bedeutung hat und

X für Wasserstoff oder Halogen, insbesondere Chlor oder Brom steht,

zunächst in einer 1. Stufe in Gegenwart einer starken Base, wie beispielsweise n-Butyllithium und in Gegenwart eines Verdünnungsmittels, wie beispielsweise Ether oder Tetrahydrofuran, zwischen −100 °C und −70 °C mit Aldehyden der Formel (V),

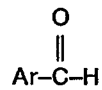 (V)

in welcher

Ar die oben angegebene Bedeutung hat, umsetzt und die so erhältlichen Carbinole der Formel (VI),

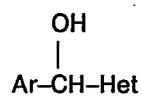 (VI)

in welcher Ar und Het die oben angegebene Bedeutung haben, in einer 2. Stufe mit einem Oxidationsmittel, wie beispielsweise Natriumdichromat, in Gegenwart eines Katalysators, wie beispielsweise Essigsäure, in allgemein üblicher Art und Weise oxidiert, und die so erhältlichen Ketone der Formel (VII),

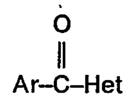 (VII)

in welcher

Ar und Het die oben angegebene Bedeutung haben,

in einer 3. Stufe mit Trimethylsulfoniumiodid der Formel (VIII),

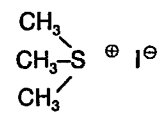 (VIII)

ebenfalls in Gegenwart einer starken Base, wie beispielsweise Kalium-tertiär-Butylat und in Gegenwart eines Verdünnungsmittels, wie beispielsweise Dimethylsulfoxid, bei Temperaturen zwischen −20 °C und +50 °C epoxidiert.

Die weiterhin zur Durchführung des erfindungsgemässen Verfahrens als Ausgangsstoffe benötigten 1,2,4-Triazol-Alkalisalze sind durch die Formel (III) allgemein definiert. In dieser Formel (III) steht $Me^{\oplus}$ vorzugsweise für ein Natrium- oder Kaliumkation.

Die Triazol-Alkalisalze der Formel (III), die Halogenheterocyclen der Formel (IV), die aromatischen Aldehyde der Formel (V) und das Trimethylsulfoniumiodid der Formel (VIII) sind allgemein bekannte Verbindungen der organischen Chemie.

Als Verdünnungsmittel zur Durchführung des erfindungsgemässen Verfahrens kommen prinzipiell inerte organische Lösungsmittel infrage. Hierzu gehören vorzugsweise Alkohole, wie beispielsweise Ethanol, Propanol oder Methoxyethanol, Ketone, wie Aceton oder Butanon, Nitrile, wie Acetonitril oder Propionitril, aromatische Kohlenwasserstoffe, wie Benzol oder Toluol, und Amide, wie beispielsweise Dimethylformamid.

Die Reaktionstemperaturen können bei der Durchführung des erfindungsgemässen Verfahrens in einem grösseren Bereich variiert werden.

Im allgemeinen arbeitet man zwischen 0 °C und +200 °C, vorzugsweise zwischen +60 °C und +150 °C.

Bei der Durchführung des erfindungsgemässen Verfahrens setzt man pro Mol Oxiran der Formel (II) im allgemeinen 1 bis 3 Mol, vorzugsweise 1 bis 2 Mol an Alkali-Salz des 1,2,4-Triazols der Formel (III) ein. Man kann das Alkalisalz des 1,2,4-Triazols der Formel (III) auch in situ erzeugen, indem man zunächst 1,2,4-Triazol und eine entsprechende Menge einer starken Alkali-Base, wie beispielsweise Natrium- oder Kaliumalkoholat oder Natrium- oder Kaliumhydrid miteinander reagieren lässt und zu diesem Reaktionsansatz das Oxiran der Formel (II) dazu gibt. Die Aufarbeitung und Isolierung der Endprodukte erfolgt nach allgemein üblichen Verfahren.

Zur Herstellung von pflanzenverträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemässen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger des Braunrostes an Weizen (Puccinia recondita) oder auch zur Bekämpfung von Pflanzenkrankheiten im Gemüsebau, wie beispielsweise gegen den Erreger des echten Gurkenmehltaus (Sphaerotheca fuliginea) einsetzen. Dabei zeigen die erfindungsgemässen Wirkstoffe auch eine hervorragende Wirkung gegen Erreger von Reiskrankheiten, wie beispielsweise gegen den Erreger der Reisfleckenkrankheit (Pyricularia oryzae), ausserdem in entsprechenden Aufwandmengen auch eine gute insektizide und pflanzenwachstumsregulierende Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei

normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:
Beispiel 1

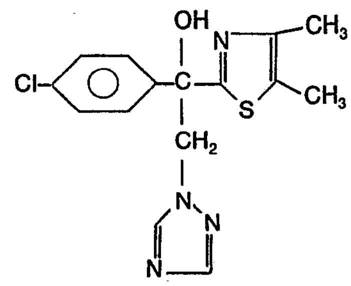

Zu einer gut gerührten Suspension von 0,9 g (0,03 Mol) 80%-igem Natriumhydrid in 40 ml Dimethylformamid fügt man portionsweise 2,1 g (0,03 Mol) 1,2,4-Triazol. (Erst nach Nachlassen der jeweiligen Wasserstoffentwicklung erfolgt eine erneute Zugabe.)

Nach beendeter Zugabe wird 30 Minuten bei Raumtemperatur nachgerührt. Danach tropft man bei Raumtemperatur eine Lösung von 8,0 g (0,03 Mol)
2-(4-Chlorphenyl)-2-(4,5-dimethylthiazol-2-yl)-oxiran
in 10 ml Dimethylformamid zu und erhitzt anschliessend 3 Stunden auf 80 °C.

Zur Aufarbeitung giesst man die erkaltete Reaktionsmischung auf ca. 200 g Eis und extrahiert mehrfach mit jeweils 50 ml Dichlormethan. Die vereinigten organischen Phasen werden mit Wasser gewaschen, über Natriumsulfat getrocknet und im Vakuum eingeengt. Der Rückstand wird aus Hexan/Tetrachlorkohlenstoff umkristallisiert. Man erhält 8,4 g (83% der Theorie) an
1-(4-Chlorphenyl)-1-(4,5-dimethylthiazol-2-yl)-2-(1,2,4-triazol-1-yl)-ethanol
vom Schmelzpunkt 174 °C.

Herstellung der Ausgangsverbindung:

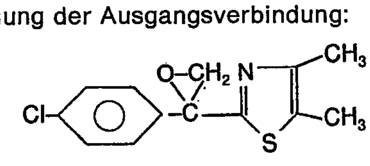

10,1 g (0,04 Mol) (4-Chlorphenyl)-(4,5-dimethylthiazol-2-yl)-methanon und 8,6 g (0,042 Mol) Trimethylsulfoniumiodid werden in 100 ml wasserfreiem Dimethylsulfoxid gelöst. Unter Rühren tropft man eine Lösung von 4,7 g (0,042 Mol) Kaliumtertiärbutanolat in 30 ml Dimethylsulfoxid innerhalb von 5 Minuten zu und rührt 20 Minuten nach, wobei die Innentemperatur 25 °C nicht übersteigen darf.

Das Gemisch wird mit 300 ml Eiswasser verdünnt, dreimal mit je 50 ml Ether extrahiert, die organische Phase mit 100 ml Wasser gewaschen und über Natriumsulfat getrocknet. Nach Abziehen des Solvens im Vakuum erhält man 9,7 g (90% der Theorie) an 2-(4-Chlorphenyl)-2-(4,5-dimethylthiazol-2-yl)-oxiran als Öl.

$^1$H-NMR (60 MHz, $CDCl_3$, ppm): ε = 2,36 (6H, s, $CH_3$ an C–4 und C–5), 3,16 3,62 (2H, d, J = 6 Hz; $CH_2$), 7,42 (4H, d, 4-Chlorphenyl-H)

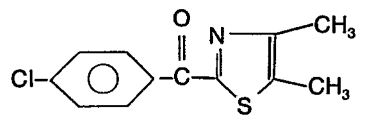

7,5 g (0,025 Mol) Natriumdichromat-dihydrat werden unter Erwärmen in 50 ml Eisessig gelöst, auf 20 °C gekühlt und mit einer Lösung von 12,7 g (0,05 Mol) (4-Chlorphenyl)-(4,5-dimethylthiazol-2-yl)-methanol in 30 ml Eisessig vermischt und anschliessend 1 Stunde unter Rückfluss erhitzt.

Nach Abkühlen auf Raumtemperatur giesst man auf 500 g Eis, extrahiert dreimal mit je 50 ml Dichlormethan und wäscht die vereinigten organischen Extrakte mit gesättigter Natriumhydrogencarbonat-Lösung und Wasser. Nach Trocknen über Natriumsulfat wird das Solvens bei vermindertem Druck abdestilliert und der Rückstand aus Ether umkristallisiert. Man erhält 11,3 g (90% der Theorie) an (4-Chlorphenyl)-(4,5-dimethylthiazol-2-yl)-methanon vom Schmelzpunkt 97 °C.

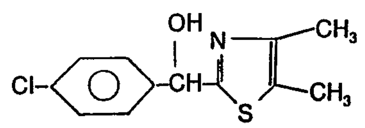

Unter Stickstoff-Atmosphäre werden zu einer Lösung von 11,5 g (0,1 Mol) 4,5-Dimethylthiazol in 100 ml wasserfreiem Ether bei −70 °C innerhalb von 30 Minuten unter Rühren 68 ml einer 1,55 molaren n-Butyllithiumlösung in n-Hexan (0,105 Mol) getropft. Man rührt bei dieser Temperatur 30 Minuten nach und gibt dann innerhalb von 20 Minuten eine Lösung von 14,0 g (0,1 Mol) 4-Chlorbenzaldehyd in 50 ml Ether zu. Dabei wird die Temperatur durch Kühlung unter −50 °C gehalten, die Suspension wird noch 30 Minuten bei Raumtemperatur nachgerührt.

Danach tropft man ca. 50 ml gesättigte Ammoniumchlorid-Lösung zu, extrahiert die wässrige Phase zweimal mit je 50 ml Ether, trocknet die vereinigten organischen Phasen über Natriumsulfat und engt im Vakuum ein. Das zurückbleibende Öl wird nach 12 Stunden fest. Durch Umkristallisation aus Petrolether (60–75 °C) erhält man 21,3 g (84% der Theorie) an (4-Chlorphenyl)-(4,5-dimethylthiazol-2-yl)-methanol vom Schmelzpunkt 107 °C.

In entsprechender Weise und gemäss den allgemeinen Herstellungsangaben erhält man die folgenden (1,2,4-Triazol-2-yl)-methylcarbinole der allgemeinen Formel (I):

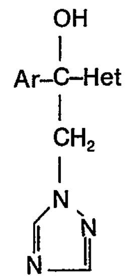

(I)

| Bsp. Nr. | Ar | Het | Physikalische Eigenschaften |
|---|---|---|---|
| 2 | 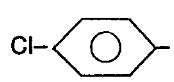 | 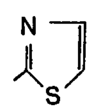 | Fp: 125 °C |
| 3 | 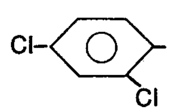 | 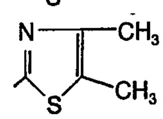 | Fp: 200 °C |
| 4 | 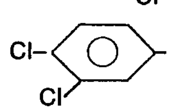 | 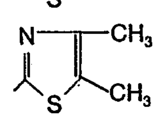 | Fp: 192 °C |
| 5 | 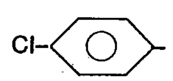 | 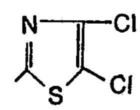 | Fp: 210 °C |
| 6 | 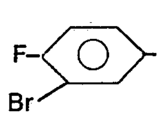 | 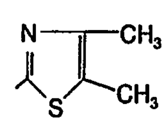 | Fp: 171 °C |
| 7 | 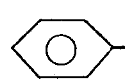 | 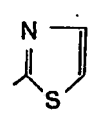 | Öl |

(Fortsetzung)

| Bsp. Nr. | Ar | Het | Physikalische Eigenschaften |
|---|---|---|---|
| 8 | 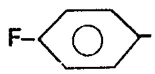 | 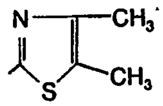 | Fp: 146 °C |
| 9 | 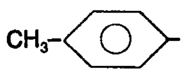 | 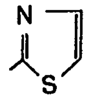 | Fp: 157 °C |
| 10 | 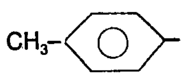 | 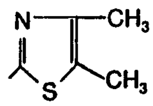 | Fp: 168 °C |
| 11 | 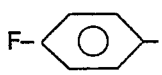 | 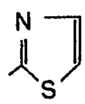 | Fp: 127 °C |

Anwendungsbeispiele:

In den folgenden Anwendungsbeispielen werden die nachstehend aufgeführten Verbindungen als Vergleichssubstanzen eingesetzt:

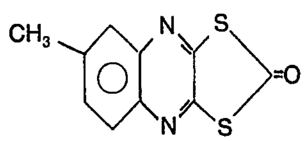

(A)

6-Methyl-2-oxo-1,3-dithiolo-[4,5-b]-chinoxalin (bekannt aus DE-B 1 100 372)

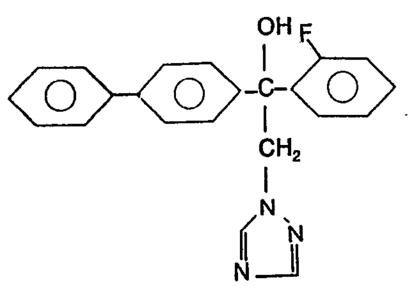

(B)

1-(4-Biphenylyl)-1-(2-fluorphenyl)-2-(1,2,4-triazol-1-yl)-ethanol

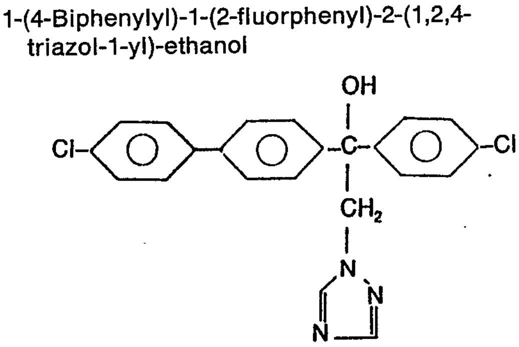

(C)

1-(4′-Chlor-4-biphenylyl)-1-(4-chlorphenyl)-2-(1,2,4-triazol-1-yl)-ethanol (bekannt aus DE-A 2 920 374).

Beispiel A

Sphaerotheca-Test (Gurke)/protektiv

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschliessend bei 23 bis 24 °C und bei einer relativen Luftfeuchtigkeit von ca. 75% im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 1, 2 und 5.

Beispiel B

Erysiphe-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 1.

Beispiel C

Puccinia-Test (Weizen)/protektiv

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässrigen Agarlösung inokuliert. Nach Antrocknen besprüht man die Pflanzen mit der Wirkstoffzubereitung taufeucht. Die Pflanzen verbleiben 24 Stunden bei 20 °C und 100% rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 2 und 3.

**Patentansprüche**

1. 1,2,4-Triazol-1-yl-methyl-carbinole der allgemeinen Formel (I),

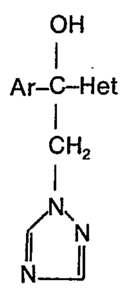 (I)

in welcher

Ar für gegebenenfalls ein- bis dreifach, gleich oder verschieden substituiertes Phenyl oder Naphthyl steht, wobei als Substituenten Fluor, Chlor, Brom, Cyano, Nitro, Methyl, Ethyl, Propyl, Butyl, Methoxy, Ethoxy, Methylthio, Trifluormethyl, Trifluormethoxy und Trifluormethylthio zu nennen sind, und

Het für einen gegebenenfalls ein- bis dreifach, gleich oder verschieden durch Methyl, Ethyl, Cyclopropyl, Cyclohexyl, Fluor, Chlor, Brom, Trifluormethyl, Chlordifluormethyl, Dichlorfluormethyl, Trichlormethyl, Dichlormethyl, Chlormethyl oder durch gegebenenfalls ein- bis dreifach substituiertes Phenyl substituierten Heterocyclus der Formel

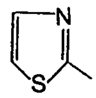 oder 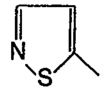

steht,

sowie deren pflanzenverträgliche Säureadditionssalze und Metallsalzkomplexe.

2. Verfahren zur Herstellung von 1,2,4-Triazol-1-yl-methyl-carbinolen der allgemeinen Formel (I),

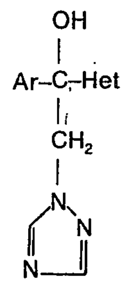 (I)

in welcher

Ar für gegebenenfalls ein- bis dreifach, gleich oder verschieden substituiertes Phenyl oder Naphthyl steht, wobei als Substituenten Fluor, Chlor, Brom, Cyano, Nitro, Methyl, Ethyl, Propyl, Butyl, Methoxy, Ethoxy, Methylthio, Trifluormethyl, Trifluormethoxy und Trifluormethylthio zu nennen sind, und

Het für einen gegebenenfalls ein- bis dreifach, gleich oder verschieden durch Methyl, Ethyl, Cyclopropyl, Cyclohexyl, Fluor, Chlor, Brom, Trifluormethyl, Chlordifluormethyl, Dichlorfluormethyl, Trichlormethyl, Dichlormethyl, Chlormethyl oder durch gegebenenfalls ein- bis dreifach substituiertes Phenyl substituierten Heterocyclus der Formel

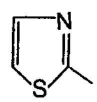 oder 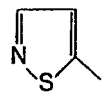

steht,

sowie von deren pflanzenverträglichen Säureadditions-Salzen und Metallsalz-Komplexen, dadurch gekennzeichnet, dass man Oxirane der Formel (II),

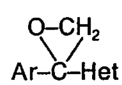 (II)

in welcher

Ar und Het die oben angegebene Bedeutung haben,

mit Alkalisalzen von 1,2,4-Triazol der Formel (III),

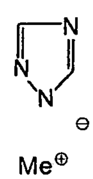 (III)

in welcher

Me$^{\oplus}$ für ein Alkalimetallkation steht, gegebenenfalls in Gegenwart eines Verdünnungsmittels umsetzt und gegebenenfalls noch an die so erhaltenen Verbindungen der Formel (I) anschliessend eine Säure oder ein Metallsalz addiert, die zu einem pflanzenverträglichen Säureadditions-Salz oder Metallsalz-Komplex führen.

3. Fungizide Mittel, gekennzeichnet durch einen Gehalt an mindestens einem 1,2,4-Triazol-1-yl-methyl-carbinol der Formel (I) gemäss Anspruch 1 bzw. einem pflanzenverträglichen Säureadditions-Salz oder Metallsalz-Komplex eines 1,2,4-Triazol-1-yl-methyl-carbinols der Formel (I).

4. Verfahren zur Bekämpfung von Pilzen, dadurch gekennzeichnet, dass man 1,2,4-Triazol-1-yl-methyl-carbinole der Formel (I) gemäss Anspruch 1 bzw. deren pflanzenverträgliche Säureadditions-Salze oder Metallsalz-Komplexe auf die Pilze oder deren Lebensraum ausbringt.

5. Verwendung von 1,2,4-Triazol-1-yl-methyl-carbinolen der Formel (I) gemäss Anspruch 1 bzw. von deren pflanzenverträglichen Säureadditions-Salzen oder Metallsalz-Komplexen als Pflanzenschutzmittel.

6. Verwendung von 1,2,4-Triazol-1-yl-methyl-carbinolen der Formel (I) gemäss Anspruch 1 bzw. von deren pflanzenverträglichen Säureadditions-Salzen oder Metallsalz-Komplexen zur Bekämpfung von Pilzen.

7. Verfahren zur Herstellung von fungiziden Mitteln, dadurch gekennzeichnet, dass man 1,2,4-Triazol-1-yl-methyl-carbinole der Formel (I) gemäss Anspruch 1 bzw. deren pflanzenverträgliche Säureadditions-Salze oder Metallsalz-Komplexe mit Streckmitteln und/oder oberflächenaktiven Stoffen vermischt.

8. Oxirane der Formel

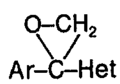

(II)

in welcher

Ar für gegebenenfalls ein- bis dreifach, gleich oder verschieden substituiertes Phenyl oder Naphthyl steht, wobei als Substituenten Fluor, Chlor, Brom, Cyano, Nitro, Methyl, Ethyl, Propyl, Butyl, Methoxy, Ethoxy, Methylthio, Trifluormethyl, Trifluormethoxy und Trifluormethylthio zu nennen sind, und

Het für einen gegebenenfalls ein- bis dreifach, gleich oder verschieden durch Methyl, Ethyl, Cyclopropyl, Cyclohexyl, Fluor, Chlor, Brom, Trifluormethyl, Chlordifluormethyl, Dichlorfluormethyl, Trichlormethyl, Dichlormethyl, Chlormethyl oder durch gegebenenfalls ein- bis dreifach substituiertes Phenyl substituierten Heterocyclus der Formel

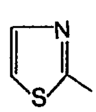 oder 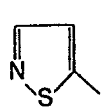

steht.

**Claims**

1. 1,2,4-Triazol-1-yl-methyl-carbinols of the general formula (I)

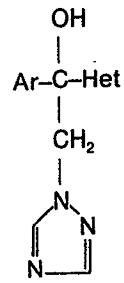

(I)

in which

Ar represents phenyl oder naphthyl, each of which is optionally monosubstituted, disubstituted or trisubstituted by identical or different substituents, substituents which may be mentioned being fluorine, chlorine, bromine, cyano, nitro, methyl, ethyl, propyl, butyl, methoxy, ethoxy, methylthio, trifluoromethyl, trifluoromethoxy and trifluoromethylthio, and represents a heterocyclic

Het ring of the formula

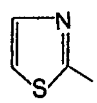 or 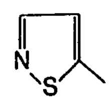

which is optionally monosubstituted, disubstituted or trisubstituted by identical or different substituents from the series comprising methyl, ethyl, cyclopropyl, cyclohexyl, fluorine, chlorine, bromine, trifluoromethyl, chlorodifluoromethyl, dichlorofluoromethyl, trichloromethyl, dichloromethyl, chloromethyl, or by optionally monosubstituted, disubstituted or trisubstituted phenyl, and acid addition salts and metal salt complexes thereof which are tolerated by plants.

2. Process for the preparation of 1,2,4-triazol-1-yl-methyl-carbinols of the general formula (I)

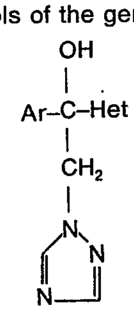

(I)

in which

Ar represents phenyl or naphthyl, each of which is optionally monosubstituted, disubstituted or trisubstituted by identical or different substituents, substituents which may be mentioned being fluorine, chlorine, bromine, cyano, nitro, methyl, ethyl, propyl, butyl, methoxy, ethoxy, methylthio, trifluoromethyl, trifluoromethoxy and trifluoromethylthio, and represents a heterocyclic

Het ring of the formula

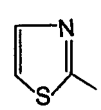 or 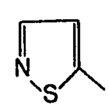

which is optionally monosubstituted, disubstituted or trisubstituted by identical or different substituents from the series comprising methyl, ethyl, cyclopropyl, cyclohexyl, fluorine, chlorine, bromine, trifluoromethyl, chlorodifluoromethyl, dichlorofluoromethyl, trichloromethyl, dichloromethyl, chloromethyl, or by optionally monosubstituted, disubstituted or trisubstituted phenyl, and of acid addition salts and metal salt complexes thereof which are tolerated by plants, characterized in that oxiranes of the formula (II)

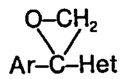

(II)

in which

Ar and Het have the abovementioned meaning, are reacted with alkali metal salts of 1,2,4-triazole of the formula (III)

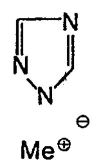

(III)

in which

$Me^{\oplus}$ represents an alkali metal cation,

if appropriate in the presence of a diluent, and, if appropriate, an acid or a metal salt resulting in an acid addition salt or metal salt complex which is tolerated by plants is then also added to the compounds of the formula (I) thus obtained.

3. Fungicidal agents, characterized in that they contain at least one 1,2,4-triazol-1-yl-methyl-carbinol of the formula (I) according to Claim 1 or an acid addition salt or metal salt complex of a 1,2,4-triazol-1-yl-methyl-carbinol, of the formula (I), which acid addition salt or metal salt complex is tolerated by plants.

4. Method of combating fungi, characterized in that 1,2,4-triazol-1-yl-methyl-carbinols of the formula (I) according to Claim 1 or acid addition salts or metal salt complexes thereof which are tolerated by plants are applied on fungi or their environment.

5. Use of 1,2,4-triazol-1-yl-methyl-carbinols of the formula (I) according to Claim 1 or acid addition salts or metal salt complexes thereof which are tolerated by plants as plant protection agents.

6. Use of 1,2,4-triazol-1-yl-methyl-carbinols of the formula (I) according to Claim 1 or acid addition salts or metal salt complexes thereof which are tolerated by plants for combating fungi.

7. Process for the preparation of fungicidal agents, characterized in that 1,2,4-triazol-1-yl-methyl-carbinols of the formula (I) according to Claim 1 or acid addition salts or metal salt complexes thereof which are tolerated by plants are mixed with extenders and/or surface-active substances.

8. Oxiranes of the formula

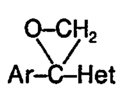

(II)

in which

Ar represents phenyl or naphthyl, each of which is optionally monosubstituted, disubstituted or trisubstituted by identical or different substituents, substituents which may be mentioned being fluorine, chlorine, bromine, cyano, nitro, methyl, ethyl, propyl, butyl, methoxy, ethoxy, methylthio, trifluoromethyl, trifluoromethoxy and trifluoromethylthio, and represents a heterocyclic

Het ring of the formula

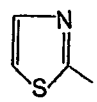

or

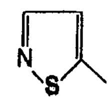

which is optionally monosubstituted, disubstituted or trisubstituted by identical or different substituents from the series comprising methyl, ethyl, cyclopropyl, cyclohexyl, fluorine, chlorine, bromine, trifluoromethyl, chlorodifluoromethyl, dichlorofluoromethyl, trichloromethyl, dichloromethyl, chloromethyl, or by optionally monosubstituted, disubstituted or trisubstituted phenyl.

**Revendications**

1. 1,2,4-triazol-1-yl-méthyl-carbinols de formule générale (I),

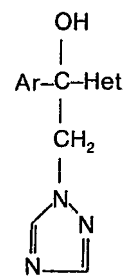

(I)

dans laquelle

Ar représente un groupe phényle ou naphtyle éventuellement substitué 1 à 3 fois, de façon identique ou différente, et l'on peut citer comme substituants un atome de fluor, de chlore ou de brome, un groupe cyano, nitro, méthyle, éthyle, propyle, butyle, méthoxy, éthoxy, méthylthio, trifluorométhyle, trifluorométhoxy et trifluorométhylthio, et

Het représente un hétérocycle de formule,

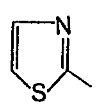

ou

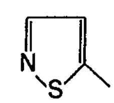

,

éventuellement substitué 1 à 3 fois, de façon identique ou différente, par un groupe méthyle, éthyle, cyclopropyle, cyclohexyle, par un atome de fluor, de chlore ou de brome, par un groupe trifluorométhyle, chlorodifluorométhyle, dichlorofluorométhyle, trichlorométhyle, dichlorométhyle, chlorométhyle ou par un groupe phényle, éventuellement substitué 1 à 3 fois,

ainsi que leurs sels d'addition d'acides et leurs complexes avec des sels de métaux, tolérables par les plantes.

2. Procédé pour préparer des 1,2,4-triazol-1-yl-méthyl-carbinols de formule générale (I),

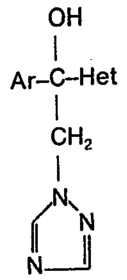

(I)

dans laquelle

Ar représente un groupe phényle ou naphtyle éventuellement substitué 1 à 3 fois, de façon identique ou différente, et l'on peut citer comme substituants un atome de fluor, de chlore, de brome, un groupe cyano, nitro, méthyle, éthyle, propyle, butyle, méthoxy, éthoxy, méthylthio, trifluorométhyle, trifluorométhoxy et trifluorométhylthio, et

Het représente un hétérocycle de formule,

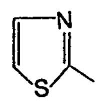

ou

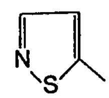

,

éventuellement substitué 1 à 3 fois, de façon identique ou différente, par un groupe méthyle, éthyle, cyclopropyle, cyclohexyle, par un atome de fluor, de chlore, de brome, par un groupe trifluorométhyle, chlorodifluorométhyle, dichlorofluorométhyle, trichlorométhyle, dichlorométhyle, chlorométhyle ou par un groupe phényle, éventuellement substitué 1 à 3 fois,

ainsi que leurs d'addition d'acides et complexes avec des sels de métaux, tolérables par les plantes, procédé caractérisé en ce que l'on fait réagir des oxirannes de formule (II),

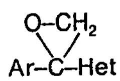

(II)

dans laquelle

Ar et Het ont le sens indiqué ci-dessus,

avec des sels alcalins du 1,2,4-triazole de formule (III),

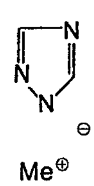

(III)

dans laquelle

Me$^{\oplus}$ représente un cation de métal alcalin,

en opérant éventuellement en présence d'un diluant, et, éventuellement, on fixe ensuite encore, sur les composés de formule (I) ainsi obtenus, par addition d'un acide ou un sel de métal, conduisant à un sel d'addition d'acide ou à un complexe de sel de métal tolérable par les plantes.

3. Produit fongicide caractérisé par une teneur en au moins un 1,2,4-triazol-1-yl-méthyl-carbinol de formule (I) selon la revendication 1 ou en un sel d'addition d'acide ou en un complexe de sel de métal d'un 1,2,4-triazol-1-yl-méthyl-carbinol de formule (I), tolérables par les plantes.

4. Procédé pour combattre des champignons, caractérisé en ce qu'on applique, sur les champignons ou sur leur biotope ou espace vital, des 1,2,4-triazol-1-yl-méthyl-carbinols de formule (I) selon la revendication 1 ou leurs sels d'addition d'acides ou complexes avec des sels de métaux, tolérables par les plantes.

5. Utilisation des 1,2,4-triazol-1-yl-méthyl-carbinols de formule (I) selon la revendication 1 ou de leurs sels d'addition d'acides ou de leurs complexes avec des sels de métaux, tolérables par les plantes, à titre de produits phytosanitaires pour la protection des plantes.

6. Utilisation des 1,2,4-triazol-1-yl-méthyl-carbinols de formule (I) selon la revendication 1, ou de leurs sels d'addition d'acides ou de leurs complexes avec des sels de métaux, tolérables par les plantes, pour combattre des champignons.

7. Procédé pour préparer des produits fongicides, caractérisé en ce qu'on mélange des 1,2,4-triazol-1-yl-méthyl-carbinols de formule (I) selon la revendication 1, ou leurs sels d'addition d'acides ou de leurs complexes avec des sels de métaux tolérables par les plantes, avec des agents d'allongement et/ou des substances tensioactives.

8. Oxirannes de formule

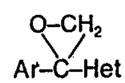

(II)

dans laquelle

Ar représente un groupe phényle ou naphtyle, substitué 1 à 3 fois, de façon identique ou différente, et l'on peut alors citer comme substituant un atome de fluor, de chlore, de brome, un groupe cyano, nitro, méthyle, éthyle, propyle, butyle, méthoxy, éthoxy, méthylthio, trifluorométhyle, trifluorométhoxy et trifluorométhylthio, et

Het représente un hétérocycle de formule

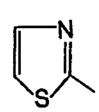

ou

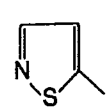

,

éventuellement substitué 1 à 3 fois, de manière identique ou différente, par un groupe méthyle, éthyle, cyclopropyle, cyclohexyle, par un atome de fluor, de chlore, de brome, par un groupe trifluorométhyle, chlorodifluorométhyle, dichlorofluorométhyle, trichlorométhyle, dichlorométhyle, chlorométhyle ou par un groupe phényle, éventuellement substitué 1 à 3 fois.